# EUROPÄISCHE PATENTSCHRIFT

(11) **EP 2 591 353 B1**
(45) Veröffentlichungstag und Bekanntmachung des Hinweises auf die Patenterteilung: **10.09.2014**
(21) Anmeldenummer: 11743186.6
(22) Anmeldetag: 07.07.2011
(51) Int. Cl.: G01N 33/38, G01N 29/28

(54) **VORRICHTUNG UND VERFAHREN ZUR IN SITU CHARAKTERISIERUNG DER QUALITÄTSPARAMETER UND/ODER DER EIGENSCHAFTEN VON ANORGANISCHEN BINDEMITTELSYSTEMEN**
DEVICE AND METHOD FOR THE IN SITU CHARACTERIZATION OF QUALITY PARAMETERS AND/OR THE PROPERTIES OF INORGANIC BINDER SYSTEMS
DISPOSITIF ET PROCÉDÉ DE CARACTÉRISATION IN SITU DES PARAMÈTRES DE QUALITÉ ET/OU DES PROPRIÉTÉS DE SYSTÈMES DE LIANTS INORGANIQUES

(30) Priorität: 20.10.2010 DE 102010060068; 08.07.2010 CH 11352010
(43) Veröffentlichungstag der Anmeldung: 15.05.2013
(73) Patentinhaber: MF Instruments GmbH, 72461 Albstadt (DE)
(72) Erfinder: TRETTIN, Reinhardt, 57250 Netphen (DE); SAKALLI, Yilmaz, 57072 Siegen (DE)
(74) Vertreter: Weiss, Peter
(86) Internationale Anmeldenummer: PCT/EP2011/003370
(87) Internationale Veröffentlichungsnummer: WO 2012/003980

(56) Entgegenhaltungen:
- WO-A1-00/34769
- H LEE: "Ultrasonic in-situ monitoring of setting process of high-performance concrete", CEMENT AND CONCRETE RESEARCH, Bd. 34, Nr. 4, 1. April 2004 (2004-04-01), Seiten 631-640, XP55006415, ISSN: 0008-8846, DOI: 10.1016/j.cemconres.2003.10.012
- REINHARDT H W ET AL: "ULTRASONIC MONITORING OF SETTING AND HARDENING OF CEMENT MORTAR - A NEW DEVICE", MATERIALS AND STRUCTURES, LONDON, GB, Bd. 33, Nr. 233, 1. November 2000 (2000-11-01), Seiten 581-583, XP009060838, ISSN: 1359-5997
- ROBEYST N ET AL: "Measuring the change in ultrasonic p-wave energy transmitted in fresh mortar with additives to monitor the setting", CEMENT AND CONCRETE RESEARCH, PERGAMON PRESS, ELMSFORD, NY, US, Bd. 39, Nr. 10, 1. Oktober 2009 (2009-10-01), Seiten 868-875, XP026563589, ISSN: 0008-8846, DOI: 10.1016/J.CEMCONRES.2009.06.016 [gefunden am 2009-07-29]
- YE: "Experimental study on ultrasonic pulse velocity evaluation of the microstructure of cementitious material at early age.", HERON, Bd. 46, Nr. 3, 1. Januar 2001 (2001-01-01) , Seite 161, XP55006417, ISSN: 0046-7316
- R. LONG: "Investigation into convenient coupling for ultrasonic transducers when inspecting concrete structures", AIP CONFERENCE PROCEEDINGS, Bd. 509, 1. Januar 2000 (2000-01-01), Seiten 1677-1684, XP55006419, ISSN: 0094-243X, DOI: 10.1063/1.1306234

## Beschreibung

Die Erfindung betrifft eine Vorrichtung zur in situ Charakterisierung der Qualitätsparameter und/oder der Eigenschaften von anorganischen Bindemittelsystemen, insbesondere von aushärtbaren Gemischen entsprechend dem Oberbegriff von Anspruch 1.

### Stand der Technik

Für die Prüfung von Qualitätsparametern und/oder von Eigenschaften von organischen Bindemittelsystemen sind aus dem Stand der Technik verschiedene Prüfverfahren bekannt. Hierbei werden insbesondere zerstörungsfreie und zerstörende Prüfverfahren unterschieden. Für Beton ist beispielsweise ein zerstörungsfreies Prüfverfahren bekannt, bei welchem Ultraschall durch den Beton geschickt wird, um die Festigkeit des Betons zu ermitteln. Dieses Verfahren macht sich zu Nutze, dass eine Laufzeit des Schalls in Beton je nach Härte des Betons variiert.

Schwierigkeiten bei dieser Messung ergeben sich aber vor allem daraus, dass der Beton beim Aushärten schrumpft bzw. schwindet. Das bedeutet, dass hierdurch zwischen der Schall-Sonde und dem Beton eine Luftstrecke erzeugt und beim Schwinden auch vergrössert wird, wodurch diese Verfahren sehr fehlerhaft sind.

Aus Reinhardt H W ET AL: "Ultrasonic Monitoring of Setting and Hardening of Cement Molar - A new Decive", Materials and Structures, London, GB, Band 33, Nr. 233, 1. November 2000 (2000-11-01, Seiten 581 bis 683, XP009060838, ISSN: 1359 bis 5997 ist eine Vorrichtung bekannt, bei der ein Mörtel in einen U-förmigen Schaumstoffbehälter eingefüllt und kompaktiert wird. Wenn der Mörtel sich verfestigt und aushärtet und das Volumen sich vermindert, kann es dazu kommen, dass der Kontakt zwischen Mörtel und Wand des Containers verloren geht. Um dieses auszugleichen, wird auf dem Mörtel eine Wasserschicht aufrecht erhalten. Dieses Wasser wird dann in einen möglichen Spalt zwischen Mörtel und der Wand des Containers einfliessen und den Kontakt sichern.

Aus H. Lee: "Ultrasonic in-situ monitoring of setting process of high-performance concrete", Cement and Concrete Research, Band 34. Nr. 4, 1. April 2004 (2004/04-01/, Seiten 631 bis 640 XP55006415, ISSN:0008-8846, DOI: 10.1016/j.cemcrones.2003.10.012" ist eine Vorrichtung zur Untersuchung von Beton bekannt, bei der zusätzlich erwähnt wird, dass auf einer Oberfläche eines Transducers ein Kopplungsmittel in ausreichender Menge vorgesehen ist.

### Aufgabe der Erfindung

Aufgabe der Erfindung ist es, eine Vorrichtung und ein Verfahren der o. g. Art zu schaffen, mit denen zerstörungsfrei In situ Charakterisierungen von anorganischen Bindemittelsystemen erfolgen, welche zuverlässig die Qualitätsparameter und die Eigenschaften ermitteln.

### Lösung der Aufgabe

Zur Lösung der Aufgabe führen die kennzeichnenden Merkmale von Anspruch 1.

Mit dieser Vorrichtung ist es möglich, in situ Qualitätsparameter und Eigenschaften eines aushärtbaren Gemisches (anorganische Bindemittelsysteme) zu charakterisieren.

Mit der erfindungsgemässen Vorrichtung und dem erfindungsgemässen Verfahren können alle möglichen Bindemittelsysteme untersucht werden. Insbesondere handelt es sich im vorliegenden Fall um Beton, Mörtel, Putze, Kleber usw.

Als Sonde kommt vor allem eine Schall-Sonde und hier eine Ultraschall-Sonde in Betracht, wobei nicht nur Schall-Laufzeitänderungen erfasst werden können, sondern auch Änderungen in der Schall-Energie und der Schall-Abschwächung oder im Frequenzspektrum. Die Erfindung soll aber nicht auf Schall-Sonden beschränkt sein, es sollen alle Sonden umfasst sein, welche ein Messmittel durch ein Bindemittelsystem schicken. Beispielsweise kann dies auch ein elektrisches Signal sein, dessen Frequenzspektrum sich je nach Dichte des Bindemittelsystems ändert. Hier soll der Erfindung keine Grenze gesetzt sein. Als Kontaktmaterial kommt vor allem ein solches in betracht, welches sich über ein längeren Zeitraum hinweg an ein Schrumpfen, Schwinden oder Ausdehnung des Bindemittelsystems anpassen kann. Hier bietet sich beispielsweise ein Gel an. Wichtig ist allein, dass dieses Gel mögliche Luftstrecken zwischen dem Bindemittelsystem und der Sonde ausgleicht. Das heisst, beim Schwinden des Bindemittels muss Gel nachfliessen, beim Ausdehnen des Bindemittels muss Gel verdrängt werden können.

In einem bevorzugten Ausführungsbeispiel soll eine Membran das Bindemittelsystem von dem Kontaktmaterial trennen. Dies muss allerdings nicht sein, das Kontaktmaterial könnte auch direkt dem Bindemittelsystem anliegen. Wird jedoch eine Membran gewählt, so muss diese elastisch sein, damit es einem Schwinden bzw. Ausdehnen des Bindemittelsystems folgen kann. Aus diesem Grund wird eine Kunststoffmembran bevorzugt, die eine Dicke von weniger als 2 mm, bevorzugt weniger als 1 mm aufweist.

Die Membran sollte bevorzugt auswechselbar ausgestaltet sein. Hierzu ist eine Platte vorgesehen, die beispielsweise in die Wand eingelassen ist. Die Platte weist eine Öffnung auf, die wiederum von der Membran überspannt ist. Wird die Platte entfernt, kann die Membran ausgewechselt oder auch abgewaschen werden.

Auch denkbar ist, dass für die Kammer selbst ein eigener Aufnahmebehälter an der Wand gebildet wird. In einem bevorzugten Ausführungsbeispiel soll die Kammer in der Wand selbst angeordnet sein. Hier ist es auch möglich, der Kammer einen Trichterschacht zuzuordnen, der ein Nachfliessen des Kontaktmaterials gewährleistet bzw. Raum für verdrängtes Kontaktmaterial bietet.

Kammer und ggf. Trichterschacht sollen bevorzugt von einem Schieber verschliessbar sein, damit nicht störende Unreinheiten in das System gelangen.

In einem bevorzugten Ausführungsbeispiel ist das Aufnahmeelement, welches das Bindemittelsystem aufnimmt, U-förmig ausgestaltet und befindet sich zwischen zwei weitgehend identisch ausgestalteten und mit je einer Sonde versehenen Wänden. Vor allem in diesem Fall wird das Aufnahmeelement aus elastischem Material, beispielsweise Schaumstoff hergestellt, wobei das Material natürlich so fest ist, dass es das Bindemittelsystem aufnehmen kann. Andererseits soll es doch so elastisch sein, dass es zwischen den beiden Wänden eingespannt werden kann.

Jeweils eine Sonde soll in einer Wand angeordnet sein. Vorzugsweise handelt es sich dabei um so genannte Sender-Empfänger-Sonden, wobei jede Sonde sowohl senden als auch empfangen kann. Denkbar ist auch, dass die Sonden mit unterschiedlichen Frequenzen arbeiten. Hierdurch können mit zwei verschiedenen Frequenzen unabhängig voneinander Messungen durchgeführt werden, welche zur gegenseitigen Überprüfung benutzt werden können. Die Messergebnisse der Sonden werden einer Auswerteeinheit zugeführt, welche dann den entsprechenden gewünschten Parameter auswertet. Wird beispielsweise die Laufzeit des Schalls gemessen, so erfolgt in der Auswerteeinheit eine Umrechnung dieser Laufzeit. Ebenfalls wird in der Auswerteeinheit ein Bezug zu beispielsweise der Festigkeit des Bindemittelsystems hergestellt.

Die beiden sich gegenüberliegenden Wände sollen bevorzugt einen festen Abstand zueinander einhalten. Hierzu sind die Wände miteinander beispielsweise über Schraubenbolzen verbunden, wobei den Schraubenbolzen im Bereich zwischen den beiden Wänden eine Distanzhülse aufgeschoben ist. Hierdurch wird ein vorbestimmter Abstand zwischen den beiden Wänden eingehalten, der auch dann aufrechterhalten bleibt, wenn das Bindemittelsystem schrumpft oder sich ausdehnt. Dies hat den Vorteil, dass keine Veränderung des Abstandes der beiden Wände vorgenommen wird, welche die Messung beeinflussen könnte. Für den Ausgleich des Abstands zwischen der Sonde und dem Bindemittelsystem sorgt das Kontaktmaterial.

Wie die Sonden in den Wänden festgelegt sind, soll von untergeordneter Bedeutung sein. Die können beispielsweise mechanisch über Schrauben fixiert werden, sie können aber auch in entsprechende Öffnungen in den Wänden eingeklebt werden. Durch den Kleber kann gleichzeitig auch eine Abdichtung erfolgen.

Im übrigen soll von der Erfindung auch umfasst sein, dass eine weitere Membran zwischen dem Kontaktmaterial und der Sonde vorgesehen ist. Diese übernimmt dann auch eine entsprechende Abdichtung, sodass die Sonde mit der Stirnfläche nicht mit dem Kontaktmaterial in Berührung kommt.

Schutz wird auch für ein Verfahren zur in situ Charakterisierung der Qualitätsparameter und/oder der Eigenschaften von anorganischen Bindemittelsystemen, insbesondere von aushärtbaren Gemischen beansprucht, wobei sich das Bindemittelsystem in einem Aufnahmeelement befindet, dem zumindest eine Wand mit einer Sonde zugeordnet wird, wobei das Bindemittelsystem in ein Aufnahmeelement eingegeben und eine Messgrösse, insbesondere eine Laufzeit von Schall, durch das Gemisch mittels der Sonde bestimmt wird, wobei ein Kontaktmaterial zwischen dem Bindemittelsystem und der zumindest einen Sonde, welches einem Schwinden oder einer Ausdehnung des Bindemittelsystems folgt, vorgesehen wird.

Das Verfahren soll vor allem anhand der Bestimmung der Laufzeit von Schall durch Beton beschrieben werden, es ist aber darauf keineswegs beschränkt. Bekannt ist, dass Beton zumindest teilweise aushärtet und dabei schwindet. Dabei fliesst immer wieder Kontaktmaterial in den Raum nach, der durch das Schwinden frei wird. Hierdurch werden Luftstrecken zwischen der Sonde und dem Bindemittelsystem verhindert. Im Laufe der Zeit des Aushärtens können in entsprechenden zeitlichen Abständen Laufzeitmessungen von Schall durch den Beton durchgeführt werden.

Vor Beginn der ersten Messung und insbesondere vor dem Einfüllen des zu untersuchenden Bindemittelsystems in das Aufnahmeelement, soll eine Kalibrierung der Vorrichtung stattfinden. Hierzu werden die beiden Wände mit ihren beiden Sonden aufeinander gelegt und die entsprechenden Kammern mit Kontaktmaterial gefüllt. Der sich hieraus ergebende Wert wird als Basis-Wert genommen. Anschiessend werden die beiden Wände über die oben erwähnten Schraubenbolzen und Distanzhülse miteinander verbunden, sodass sie einen bestimmten Abstand voneinander aufweisen. Nach Einfüllen des Bindemittelsystems in das Aufnahmeelement sind nun kalibrierte Messungen möglich.

Die erfindungsgemässe Vorrichtung soll sowohl im Rahmen der Qualitätssicherung als auch im Rahmen der Materialcharakterisierung eingesetzt werden. Mit dieser Vorrichtung kann nicht nur die Druckfestigkeit ermittelt werden, sondern auch der Hydratationsverlauf, die Reaktionskinetik, das Gefüge (Mikro- und Nanogefüge), die mechanischen Eigenschaften (Biege-und Zugfestigkeit), die Bildung der Hydratationsprodukte und die Porosität charakterisiert werden. Erfasst werden vor allem Schallaufzeitänderungen, Schallenergieänderungen, die Änderungen des Frequenzspektrums und die Änderung der Schallabschwächung.

### FIGURENBESCHREIBUNG

Nachfolgend wird die Erfindung anhand der beiliegenden Figuren kurz beschrieben, wobei die Figuren zeigen:
- **Figur 1**: eine Draufsicht auf eine erfindungsgemässe Vorrichtung zur in situ Charakterisierung der Qualitätsparameter und der Eigenschaften von anorganischen Bindemittelsystemen;
- **Figur 2**: eine Seitenansicht auf eine Wand der Vorrichtung gemäss Figur 1.

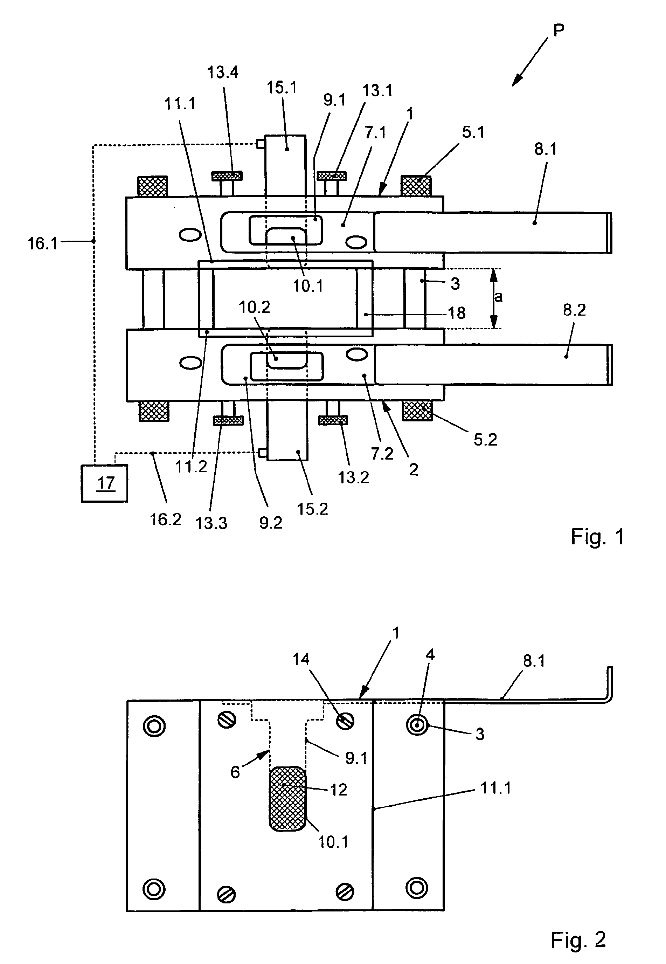

Eine erfindungsgemässe Vorrichtung P zur in situ Charakterisierung der Qualitätsparameter und der Eigenschaften von anorganischen Bindemittelsystemen weist gemäss Figur 1 zwei Wände 1 und 2 auf, die in einem Abstand a voneinander angeordnet sind. Dieser Abstand a wird von Distanzhülsen 3 bestimmt, die jeweils einem Schraubenbolzen 4 aufgeschoben sind. Vier derartige Schraubenbolzen 4 durchsetzen jeweils die Wände 1 und 2, wobei auf aus den Wänden 1 und 2 (nicht mehr dargestellte) herausragende Enden des Schraubenbolzens 4 Rändelmuttern 5.1 bzw. 5.2 aufgeschraubt sind.

Jede Wand 1 bzw. 2 besitzt eine in Figur 2 gestrichelt bzw. erkennbare Stufenausnehmung 6. Ein von oben gesehen erster Teil der Stufenausnehmung 6 ist als eine flache Führung 7.1 bzw. 7.2 für einen Schieber 8.1 bzw. 8.2 ausgebildet. Mit diesem Schieber 8.1 bzw. 8.2 kann die Stufenausnehmung 6 insgesamt verschlossen werden.

An die flache Führung 7.1 bzw. 7.2 schliesst ein Trichterschacht 9.1 bzw. 9.2 an, der in eine ovale Kammer 10.1 bzw. 10.2 übergeht. Dies ovale Kammer 10.1 bzw. 10.2 durchbricht in dieser ovalen Form auch die Wand 1 bzw. 2 nach aussen hin und sogar eine Platte 11.1 bzw. 11.2, welche in die Wand 1 bzw. 2 eingelassen ist. Allerdings wird die Kammer 10.1 bzw. 10.2 von einer Membran 12 überdeckt, welche zwischen der Platte 11.1 bzw. 11.2 und der Wand 1 bzw. 2 eingespannt ist. Diese Membran 12 kann bei Bedarf auch ausgetauscht werden, wozu vier Rändelmuttern 13.1 - 13.4 gelöst werden, welche auf Schraubenbolzen 14 aufsitzen, die wiederum durch die Wand 1 bzw. 2 hindurchgeführt sind.

An die Kammer 10.1 bzw. 10.2 schliesst eine Schall-Sonde 15.1 bzw.15.2 an, die über eine entsprechende Verbindung 16.1 bzw. 16.2 mit einer Auswerteeinheit 17 in Verbindung steht.

Zwischen den beiden Wänden 1 und 2 ist ein U-förmiges Aufnahmeelement 18 eingesetzt, welches bevorzugt aus einem Schaumstoff besteht. Dieses Aufnahmeelement 18 dient der Aufnahme eines zu untersuchenden Bindemittelsystems, weshalb es von der Festigkeit her geeignet sein muss, dieses Bindemittelsystem aufzunehmen und zu halten. Andererseits muss es elastisch genug sein, damit es abdichtend zwischen den beiden Wänden 1 und 2 eingespannt werden kann. Aus diesem Grunde muss auch eine Breite des Aufnahmeelementes 18 mit einer Länge der Distanzhülse 3 abgestimmt werden, sodass es zu einem geeigneten Einspannen des Aufnahmeelementes 18 zwischen den beiden Wänden 1 und 2 kommt.

Die Funktionsweise der vorliegenden Erfindung ist folgende:

Zunächst werden die beiden Wände 1 und 2 aufeinander festgelegt, um die Schall-Sonden 15.1 und 15.2 zu kalibrieren und die Auswerteeinheit 17 mit den entsprechenden Signalen zu füttern. Die beiden Kammern 10.1 und 10.2 sind mit Gel gefüllt und die entsprechenden Stufenausnehmungen 6.1 bzw. 6.2 durch die Schieber 8.1 bzw. 8.2 verschlossen.

Nach dieser Kalibrierung werden die beiden Wände 1 und 2 durch die entsprechenden Schraubenbolzen 4 verbunden und der Abstand a durch die Distanzhülsen 3 erzeugt. Dabei wird bereits das Aufnahmeelement 18 zwischen die beiden Wände 1 und 2 eingesetzt bzw. eingespannt.

Nunmehr kann ein zu untersuchendes Bindemittelsystem in das Aufnahmeelement 18 eingefüllt werden. Dieses Bindemittelsystem härtet aus, wobei nach vorgegebenen Zeitspannen Messungen mittels der Schall-Sonden 15.1 und 15.2 durchgeführt werden, um beispielsweise eine erreichte Druckfestigkeit des Bindemittelsystems in dem Aufnahmeelement 18 zu überprüfen. Im Rahmen der Erfindung ist es dabei wesentlich, dass ein Schwinden des Bindemittelsystems in dem Aufnahmeelement 18 durch Nachfliessen von Gel ausgeglichen wird, welches die Membran 12.1 bzw. 12.2 gegen das schwindende Bindemittelsystem drückt. Der Trichterschacht 9.1 bzw. 9.2 dient dabei als Vorratsraum für das nachfliessende Gel.

Die Membranen 12.1 bzw. 12.2 bestehen bevorzugt aus einem Kunststoff und besitzen eine Dicke von weniger als 2 mm, bevorzugt von weniger als 1 mm. Beim Schwinden des Bindemittelsystems folgen die Membranen unter dem Druck des Gels, sodass sie permanent in Kontakt mit dem Bindemittelsystem bleiben. Hierdurch wird eine Übertragung der Schall-Sonden 15.1 bzw. 15.2 nicht unterbrochen, das heisst, es befindet sich weder Luft zwischen der Membran und dem zu untersuchenden Bindemittelsystems noch zwischen der Membran und dem Gel.

Das erfindungsgemässe Verfahren kann z. Bsp. bei der Untersuchung der Druckfestigkeit von Beton angewendet werden. Nach der oben beschriebenen Kalibrierung der Schall-Sonden 15.1 und 15.2 und dem Abgleich durch die Auswerteeinheit 17, wird Beton in das Aufnahmeelement 18 gefüllt. Danach wird 28 Tage abgewartet, um die 28-Tage-Druckfestigkeit des Betons durch Laufzeitmessung von Schall und durch Auswertung dieser Laufzeitmessung in der Auswerteeinheit 17 zu ermitteln. Nach einer vorgegebenen Zeitspanne, beispielsweise einem Jahr, kann nochmals eine Messung durchgeführt werden, um eine weitere Druckfestigkeit zu ermitteln. Das Schwinden des Betons wird durch Nachfliessen von Gel und dem Nachdrücken der Membran 12. 1 bzw. 12.2 beim Schwinden des Betons ausgeglichen.

**Bezugszeichenliste**

| | | | | | |
|---|---|---|---|---|---|
| 1 | Wand | 34 | | 67 | |
| 2 | Wand | 35 | | 68 | |
| 3 | Distanzhülse | 36 | | 69 | |
| 4 | Schraubenbolzen | 37 | | 70 | |
| 5 | Rändelmutter | 38 | | 71 | |
| 6 | Stufenausnehmung | 39 | | 72 | |
| 7 | Führung | 40 | | 73 | |
| 8 | Schieber | 41 | | 74 | |
| 9 | Trichterschacht | 42 | | 75 | |
| 10 | Kammer | 43 | | 76 | |
| 11 | Platte | 44 | | 77 | |
| 12 | Membran | 45 | | 78 | |
| 13 | Rändelmutter | 46 | | 79 | |
| 14 | Schraubenbolzen | 47 | | | |
| 15 | Schall-Sonde | 48 | | | |
| 16 | Verbindung | 49 | | | |
| 17 | Auswerteeinheit | 50 | | a | Abstand |
| 18 | Aufnahmeelement | 51 | | P | Vorrichtung |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung zur in situ Charakterisierung der Qualitätsparameter und/oder der Eigenschaften von anorganischen Bindemittelsystemen, insbesondere von aushärtbaren Gemischen, wobei sich das Bindemittelsystem in einem Aufnahmeelement (18) befindet, dem zumindest eine Wand (1, 2) mit einer Sonde (15.1, 15.2) zugeordnet ist, und ein Kontaktmaterial zum Ausgleich von möglichen Luftstrecken zwischen dem Bindemittelsystem und der Sonde vorgesehen ist,
**dadurch gekennzeichnet,**
**dass** zwischen dem Bindemittelsystem und der Sonde (15.1, 15.2) eine Kammer (10.1, 10.2) für ein sich einem Schrumpfen, Schwinden oder Ausdehnen des Bindemittelsystems anpassendes Kontaktmaterial vorgesehen ist.

2. Vorrichtung nach Anspruch 1, **dadurch gekennzeichnet, dass** eine Membran (12.1, 12.2) das Bindemittelsystem von dem Kontaktmaterial trennt.

3. Vorrichtung nach Anspruch 2, **dadurch gekennzeichnet, dass** die Membran (12.1, 12.2) zwischen einer Platte (11.1, 11.2) und der Wand (1, 2) angeordnet und diese gegebenenfalls in die Wand (1, 2) eingelassen ist.

4. Vorrichtung nach Anspruch 3, **dadurch gekennzeichnet, dass** die Platte (11.1, 11.2) entfernbar mit der Wand (1, 2) verbunden ist.

5. Vorrichtung nach einem der vorhergehenden Ansprüchen, **dadurch gekennzeichnet, dass** die Kammer (10.1, 10.2) in der Wand (1, 2) angeordnet ist.

6. Vorrichtung nach einem der vorhergehenden Ansprüchen, **dadurch gekennzeichnet, dass** in die Kammer (10.1, 10.2) ein Trichterschacht (9.1, 9.2) einmündet.

7. Vorrichtung nach einem der vorhergehenden Ansprüchen, **dadurch gekennzeichnet, dass** die Vorrichtung einen Schieber (8.1, 8.2) umfasst, mit dem die Kammer (10.1, 10.2) bzw. der Trichterschacht (9,1, 9.2) verschliessbar ist.

8. Vorrichtung nach einem der vorhergehenden Ansprüche, **dadurch gekennzeichnet, dass** in der Wand (1, 2) die Sonde (15.1, 15.2) der Kammer (10.1, 10 2) zugeordnet ist.

9. Vorrichtung nach einem der vorhergehenden Ansprüche, **dadurch gekennzeichnet, dass** das Aufnahmeelement (18) U-förmig ausgestaltet und zwischen zwei weitgehend identisch ausgestalteten und mit je einer Sonde (15.1, 15.2) versehenen Wänden (1, 2) angeordnet ist.

10. Vorrichtung nach Anspruch 9, **dadurch gekennzeichnet, dass** die zwei sich gegenüberliegenden Wände (1, 2) einen Abstand (a) zueinander einhalten.

11. Vorrichtung nach Anspruch 9 oder 10, **dadurch gekennzeichnet, dass** die Wände (1, 2) über Bolzen (4) mit aufgeschobenen Distanzhülsen (3) miteinander verbunden sind.

12. Vorrichtung nach einem der vorhergehenden Ansprüche, **dadurch gekennzeichnet, dass** das Aufnahmeelement (18) flexibel, insbesondere aus Kunststoff ist.

13. Vorrichtung nach einem der vorhergehenden Ansprüche, **dadurch gekennzeichnet, dass** die Sonde eine Schall-Sonde (15.1, 15.2) ist.

## Claims

1. A device for the in situ characterisation of the quality parameters and/or the properties of inorganic binder systems, in particular of curable mixtures, wherein the binder system is located in a receiving element (18) with which at least one wall (1, 2) with a probe (15.1, 15.2) is associated, and a contact material for compensating for possible air gaps between the binder system and the probe is provided,
**characterised in that**
a chamber (10.1, 10.2) for a contact material which adapts to shrinkage, contraction or expansion of the binder system is provided between the binder system and the probe (15.1, 15.2).

2. A device according to Claim 1, **characterised in that** a membrane (12.1, 12.2) separates the binder system from the contact material.

3. A device according to Claim 2, **characterised in that** the membrane (12.1, 12.2) is arranged between a plate (11.1, 11.2) and the wall (1, 2), and said plate is optionally set into the wall (1, 2).

4. A device according to Claim 3, **characterised in that** the plate (11.1, 11.2) is connected removably to the wall (1, 2).

5. A device according to one of the preceding claims, **characterised in that** the chamber (10.1, 10.2) is arranged in the wall (1, 2).

6. A device according to one of the preceding claims, **characterised in that** a hopper shaft (9.1, 9.2) opens into the chamber (10.1, 10.2).

7. A device according to one of the preceding claims, **characterised in that** the device comprises a slider (8.1, 8.2) with which the chamber (10.1, 10.2) or the hopper shaft (9.1, 9.2) can be closed.

8. A device according to one of the preceding claims, **characterised in that** the probe (15.1, 15.2) is associated with the chamber (10.1, 10.2) in the wall (1, 2).

9. A device according to one of the preceding claims, **characterised in that** the receiving element (18) is U-shaped and is arranged between two largely identically configured walls (1, 2) which are provided with one probe (15.1, 15.2) each.

10. A device according to Claim 9, **characterised in that** the two opposing walls (1, 2) maintain a distance (a) from each other.

11. A device according to Claim 9 or 10, **characterised in that** the walls (1, 2) are connected together via bolts (4) with pushed-on spacer sleeves (3).

12. A device according to one of the preceding claims, **characterised in that** the receiving element (18) is flexible, in particular is made of plastics material.

13. A device according to one of the preceding claims, **characterised in that** the probe is a sound probe (15.1, 15.2).

## Revendications

1. Dispositif de caractérisation in situ des paramètres de qualité et/ou des propriétés de systèmes de liants inorganiques, en particulier de mélanges durcissables, le système de liants se trouvant dans un élément de réception (18) auquel est associé au moins une paroi (1, 2) avec une sonde (15.1, 15.2), et un matériau de contact pour compenser de possibles trajets d'air étant prévu entre le système de liants et la sonde,
**caractérisé par le fait**
**qu'**entre le système de liants et la sonde (15.1, 15.2) est prévue une chambre (10.1, 10.2) pour un matériau de contact s'adaptant à la rétraction, au rétrécissement ou à l'expansion du système de liants.

2. Dispositif selon la revendication 1, **caractérisé par le fait qu'**une membrane (12.1, 12.2) sépare le système de liants du matériau de contact.

3. Appareil selon la revendication 2, **caractérisé par le fait que** la membrane (12.1, 12.2) est disposée entre une plaque (11.1, 11.2) et la paroi (1, 2) et qu'elle est éventuellement incorporée dans la paroi (1, 2).

4. Appareil selon la revendication 3, **caractérisé par le fait que** la plaque (11.1, 11.2) est connectée de manière amovible à la paroi (1, 2).

5. Dispositif selon l'une des revendications précédentes, **caractérisé par le fait que** la chambre (10.1, 10.2) est disposé dans la paroi (1, 2).

6. Dispositif selon l'une des revendications précédentes, **caractérisé par le fait que** dans la chambre (10.1, 10.2) aboutit un puits de trémie (9.1, 9.2).

7. Dispositif selon l'une des revendications précédentes, **caractérisé par le fait que** le dispositif comprend un registre (8.1, 8.2) par lequel la chambre (10.1, 10.2) ou le puits de trémie (9.1, 9.2) peut être fermé.

8. Dispositif selon l'une des revendications précédentes, **caractérisé par le fait que** dans la paroi (1, 2) la sonde (15.1, 15.2) est associée à la chambre (10.1, 10.2).

9. Dispositif selon l'une des revendications précédentes, **caractérisé par le fait que** l'élément de réception (18) est réalisé en forme de "U" et disposé entre deux parois (1, 2) réalisées largement identiques et pourvues, chacune, d'une sonde (15.1, 15.2).

10. Dispositif selon la revendication 9, **caractérisé par le fait que** les deux parois opposées (1, 2) maintiennent une distance (a) entre elles.

11. Dispositif selon la revendication 9 ou 10, **caractérisé par le fait que** les parois (1, 2) sont connectées entre elles par l'intermédiaire de boulons (4) avec des douilles d'écartement (3) y enfilées.

12. Dispositif selon l'une des revendications précédentes, **caractérisé par le fait que** l'élément de réception (18) est flexible, en particulier réalisé en matière plastique.

13. Dispositif selon l'une des revendications précédentes, **caractérisé par le fait que** ls sonde est une sonde sonore (15.1, 15.2).
